# EUROPÄISCHE PATENTSCHRIFT

(11) **EP 0 594 574 B1**
(45) Veröffentlichungstag und Bekanntmachung des Hinweises auf die Patenterteilung: **21.06.1995**
(21) Anmeldenummer: 91900255.0
(22) Anmeldetag: 07.12.1990
(51) Int. Cl.: C07C 303/06, C11D 1/28

(54) **VERFAHREN ZUR HERSTELLUNG VON SALZEN SULFIERTER FETTSÄUREGLYCERINESTER**
PROCESS FOR MAKING SALTS OF SULPHONATED FATTY ACID GLYCERINE ESTERS
PROCEDE DE FABRICATION DE SELS D'ESTERS SULFONES D'ACIDES GRAS ET DE GLYCERINE

(30) Priorität: 15.12.1989 DE 3941365
(43) Veröffentlichungstag der Anmeldung: 04.05.1994
(73) Patentinhaber: Henkel Kommanditgesellschaft auf Aktien, 40191 Düsseldorf (DE)
(72) Erfinder: FABRY, Bernd, Dr., D-4052 Korschenbroich (DE); WANGEMANN, Frank, Dr., D-4020 Mettmann (DE); SCHÄFER, Michael, D-4006 Erkrath 1 (DE)
(86) Internationale Anmeldenummer: EP9002130
(87) Internationale Veröffentlichungsnummer: WO9109009

(56) Entgegenhaltungen:
- EP-A- 0 130 753
- EP-A- 0 178 557
- EP-A- 0 334 199
- EP-A- 0 353 503
- EP-A- 0 371 369

## Beschreibung

Gegenstand der Erfindung ist ein Verfahren zur Herstellung von wässrigen Lösungen von Alkali-, Erdalkali-, Ammonium- und/oder Aminsalzen sulfierter Fettsäureglycerinester durch Umsetzung von Fettsäureglycerinestern mit Iodzahlen kleiner 5 mit gasförmigem Schwefeltrioxid in Reaktoren, die nach dem kontinuierlichen Fallfilmprinzip arbeiten, und anschließender Neutralisation mit wässrigen Basen sowie die Verwendung der Sulfierprodukte zur Herstellung oberflächenaktiver Mittel.

Sulfierprodukte gesättigter Fettsäureglycerinester haben Bedeutung als Hilfsmittel in der Textil-, Faser- und Ledertechnik. Zu ihrer Herstellung geht man gewöhnlich von pflanzlichen oder tierischen Fetten niedriger Iodzahl aus, die mit Schwefelsäure oder Oleum umgesetzt werden. Der schwerwiegende technische Nachteil dieses Verfahrens liegt jedoch darin, daß im Anschluß an die Sulfierung nicht nur die gebildete Sulfonsäure, sondern auch die als Lösungsmittel dienende Schwefelsäure neutralisiert werden muß, was zu einer unerwünscht hohen Elektrolytbelastung der Produkte und zu unvorteilhaften anwendungstechnischen Eigenschaften, so z. B. im Hinblick auf das Korrosionsverhalten führt.

Es hat in der Vergangenheit daher nicht an Versuchen gemangelt, das Problem der Sulfierung von gesättigten Fettsäureglycerinestern auf anderem Wege zu lösen. Aus den deutschen Patentschriften **DE 1 186 051** und **DE 1 443 992** sind z. B. zwei Verfahren bekannt, nach denen Gemische aus gesättigten Triglyceriden und niederen Alkylestern einer gemeinsamen, diskontinuierlichen Umsetzung mit gasförmigem Schwefeltrioxid unterworfen werden. Die dabei anfallenden Sulfierprodukte enthalten neben sulfierten Triglyceriden, alpha-Sulfofettsäure-alkylester und weisen ein anwendungstechnisches Profil auf, das sich von den Produkten, die durch Oleumsulfierung von Triglyceriden gewonnen werden, unvorteilhaft unterscheidet. Ausdrücklich wird in den Patentschriften darauf hingewiesen, daß eine direkte Gassulfierung gesättigter Trigly- ceride infolge einer zu hohen Viskosität der Sulfierprodukte und starker Schaumentwicklung nicht möglich ist.

Das zu lösende technische Problem bestand somit darin, ein Verfahren zu entwickeln, das die Direktsulfierung gesättigter Triglyceride mit gasförmigem Schwefeltrioxid gestattet und nicht mit den geschilderten Nachteilen behaftet ist.

Gegenstand der Erfindung ist ein Verfahren zur Herstellung von wässrigen Lösungen von Alkali-, Erdalkali-, Ammonium- und/oder Aminsalzen sulfierter Fettsäureglycerinester durch Umsetzung von Fettsäureglycerinestern mit Iodzahlen kleiner 5 mit gasförmigem Schwefeltrioxid in Reaktoren, die nach dem kontinuierlichen Fallfilmprinzip arbeiten, und anschließende Neutralisation mit wässrigen Basen.

Unter Fettsäureglycerinester sind die Mono-, Di- und Triester sowie deren Gemische zu verstehen, wie sie bei der Herstellung durch Veresterung von 1 Mol Glycerin mit 1 bis 3 Mol Fettsäure oder bei der Umesterung von Triglyceriden mit 0.5 bis 2 Mol Glycerin erhalten werden. Insbesondere werden diejenigen Fettsäureglycerinester eingesetzt, die sich von gesättigten Fettsäuren mit 8 bis 22 Kohlenstoffatomen ableiten, so z. B. Capronsäure, Caprylsäure, Caprinsäure, Myristinsäure, Laurinsäure, Palmitinsäure, Stearinsäure oder Behensäure.

Geht man von Fetten und Ölen, also natürlichen Gemischen unterschiedlicher Fettsäureglycerinester aus, ist es erforderlich, die Einsatzprodukte vor der Sulfierung in an sich bekannter Weise mit Wasserstoff weitgehend abzusättigen, d. h. auf Iodzahlen kleiner 5, vorteilhafterweise kleiner 2 zu härten, da andernfalls Sulfierprodukte unzureichender Farbqualität resultieren. Typische Beispiele geeigneter Einsatzstoffe sind Palmöl, Palmkernöl, Olivenöl, Rüböl, Korianderöl, Sonnenblumenöl, Baumwollsaatöl, Erdnußöl, Leinöl, Lardöl oder Schweineschmalz. Aufgrund ihres hohen natürlichen Anteils an gesättigten Fettsäuren hat es sich jedoch als besonders vorteilhaft erwiesen, von Kokosöl oder Rindertalg auszugehen.

Die Umsetzung von Fettsäureglycerinestern mit Iodzahlen kleiner 5 mit gasförmigem Schwefeltrioxid erfolgt in Reaktoren, die nach dem kontinuierlichen Fallfilmprinzip arbeiten. Das wesentliche Merkmal dieses Typs von Reaktoren besteht darin, daß das zu sulfierende Einsatzmaterial mit Hilfe geeigneter apparativer Maßnahmen, z. B. durch eine Düse oder einen Überlauf, dergestalt in den Reaktor eingebracht wird, daß es entlang der Rohrwandung als dünner Film abläuft, während die Begasung mit Schwefeltrioxid wahlweise horizontal oder vertikal dazu erfolgen kann. Infolge des raschen Stoffaustausches und einer großen, der Kühlung zur Verfügung stehenden Oberfläche verläuft die Sulfierung in kontinuierlich arbeitenden Fallfilmreaktoren unter schonenden Bedingungen ab und führt im allgemeinen zu besonders hellfarbigen Produkten. Reaktoren dieses Types sind z. B. aus der DE 2 138 038 bekannt; eine zusammenfassende Beschreibung findet sich ferner in J.Falbe (Ed.), "Surfactants in Consumer Products", Springer Verlag, Berlin, 1987, S.61f. Neben diesem an sich bekannten Vorteil des kontinuierlich arbeitenden Fallfilmreaktors wurde jedoch überraschenderweise gefunden, daß auch Stoff, wie z. B. gesättigte Fettsäureglycerinester mit gasförmigem Schwefeltrioxid umgesetzt werden können, bei denen eine diskontinuierliche Sulfierung, z. B. im Rührkessel, infolge von Viskositäts- und Schaumproblemen nicht möglich ist.

Zur Gassulfierung im kontinuierlich arbeitenden Fallfilreaktor wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt.

Das Einsatzverhältnis des gasförmigen Schwefeltrioxids beträgt 0.5 bis 5.0 Mol pro Mol Fettsäureglycerinester. Vorteilhaft ist es jedoch, Einsatzverhältnisse von 2.0 bis 3.0 Mol SO₃ pro Mol Ester zu wählen, da bei niedrigeren Konzentrationen des Sulfieragens Produkte mit unzureichenden oberflächenaktiven Eigenschaften erhalten werden und im höheren Konzentrationsbereich eine unbefriedigende SO₃-Aufnahme durch den Ester beobachtet wird.

Die Sulfierreaktion wird bei Temperaturen zwischen 25 und 98°C durchgeführt. Da die Einsatzstoffe bei Raumtemperatur in der Regel als Feststoffe oder zähe Flüssigkeiten vorliegen, empfiehlt es sich jedoch, Reaktionstemperaturen zwischen 50 und 85°C zu wählen, da auf diese Weise sowohl eine ausreichende Pumpbarkeit als auch die Ausbildung eines dünnen Fallfilms im Reaktor gewährleistet wird.

Die bei der Sulfierung anfallenden sauren Sulfierprodukte werden mit wässrigen Basen neutralisiert, wobei der pH-Wert zwischen 6.5 und 7.5 gehalten wird. Als Basen für die Neutralisation kommen Alkalimetallhydroxide, wie Natrium-, Kalium- und Lithiumhydroxid, Erdalkalimetalloxide und -hydroxide wie Magnesiumoxid, Calciumoxid und Calciunhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine in Betracht. Die Neutralisationsbasen gelangen dabei insbesondere in Form 25 bis 50 gew.- %iger wässriger Lösungen zum Einsatz, wobei Natriumhydroxidlösungen bevorzugt sind.

In einer bevorzugten Ausführungsform des erfindungsgemäßen Verfahrens können die sauren Sulfierprodukte vor der Neutralisation einer zusätzlichen Nachreaktion über einen Zeitraum von 5 bis 60, vorzugsweise 15 bis 30 min und bei einer Temperatur von 25 bis 90, vorzugsweise 50 bis 80°C unterworfen werden. Auf diesem Wege kann eine Verminderung der unsulfierten Anteile erzielt werden.

Das Sulfierprodukt stellt ein komplexes Gemisch dar, das im wesentlichen Mono-, Di- und Triglyceridsulfonate mit alpha-ständiger Sulfonsäuregruppierung enthält. Als Nebenprodukte bilden sich alpha-sulfonierte Fettsäuren, Glyceridsulfate, Glycerinsulfate, Glycerin und Seifen.

Die Sulfierprodukte liegen nach Neutralisation als hellfarbige, cremige Pasten vor, so daß eine Bleiche in der Regel nicht erforderlich ist. Dennoch kann durch Zusatz von Wasserstoffperoxid- oder Natriumhypochloritlösung in an sich bekannter Weise eine weitere Aufhellung der Produkte erzielt werden. Dabei werden, bezogen auf den Feststoffgehalt in der Lösung der Sulfierprodukte, 0.2 bis 2.0 Gew.-% Wasserstoffperoxid, berechnet als 100 gew.-%ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

Die Produkte zeichnen sich durch eine deutliche Erniedrigung der Oberflächenspannung des Wassers aus und fördern die Benetzung fester Grenzflächen mit Wasser. Ferner eignen sie sich zur Emulgierung von Fetten und Ölen in wässrigen Systemen.

Die Erfindung betrifft daher ferner die Verwendung der nach dem geschilderten Verfahren erhältlichen Umsetzungsprodukte von Fettsäureglycerinestern mit Iodzahlen kleiner 5 mit gasförmigem Schwefeltrioxid zur Herstellung oberflächenaktiver Mittel.

Die folgenden Beispiele sollen den Gegenstand der Erfindung näher erläutern.

### Beispiele

**Tab.1**

| Durchschnittliche Zusammensetzung der eingesetzten Triglyceride (Edukte) nach Härtung; Prozentangaben in Gew.-% | | | | | |
|---|---|---|---|---|---|
| Fettsäure | HK % | HPK % | HP % | HR % | HRT % |
| Capronsäure | 1 | 1 | - | - | - |
| Caprylsäure | 8 | 4 | - | - | - |
| Caprinsäure | 7 | 5 | - | - | - |
| Laurinsäure | 48 | 50 | 2 | - | - |
| Myristinsäure | 17 | 15 | 42 | 1 | 3 |
| Palmitinsäure | 9 | 7 | 56 | 4 | 25 |
| Stearinsäure | 10 | 18 | - | 95 | 72 |
| Iodzahl | 2 | 2 | 2 | 2 | 1 |
| Legende: HK = Gehärtetes Kokosöl HPK = Gehärtetes Palmkernöl HP = Gehärtetes Palmöl HR = Gehärtetes Neues Rüböl HRT = Gehärteter Rindertalg | | | | | |

### Beispiele 1 - 5:

**Allgemeine Arbeitsvorschrift zur Kontisulfierung von gesättigten Fettsäureglycerinestern.** In einem kontinuierlich arbeitenden Fallfilmreaktor (Länge 120 cm, Querschnitt 1 cm, Eduktdurchsatz 600 g/h) mit Mantelkühlung und seitlicher SO₃-Begasung wurden 5 Mol eines Fettsäureglycerinesters mit einer Iodzahl kleiner 5 bei T = 30 bis 80°C mit 5.0 bis 15.0 Mol gasförmigem Schwefeltrioxid zur Reaktion gebracht. Der Fettsäureglycerinester wurde dabei über eine Düse mit einer Öffnung von 0.2 cm derartig in den Reaktor gesprüht, daß das Edukt entlang der Rohrwandung einen kontinuierlichen feinen Film mit einer Schichtdicke von weniger als 0.1 cm bildete. Das Schwefeltrioxid wurde durch Erhitzen aus einer entsprechenden Menge 65 gew.-%igen Oleums ausgetrieben, mit Stickstoff auf eine Konzentration von 2 Vol.-% verdünnt und am Reaktorkopf seitlich eingeblasen. Anschließend wurde das saure Sulfierprodukt kontinuierlich zusammen mit wässriger, 25 gew.-%iger Natronlauge bei pH 6.5 bis 7.5 neutralisiert. Die Kenndaten der Beispiele sind in Tab.2 zusammengefaßt.

Der Aniontensidgehalt (WAS) sowie die Unsulfierten Anteile (US) wurden nach den DGF-Einheitsmethoden, Stuttgart 1950-1984, H-III-10 und G-II-6b ermittelt. Der Sulfatgehalt wurde als Natriumsulfat berechnet, die Bestimmung des Wassergehaltes erfolgte nach der Fischer-Methode.
Die Bestimmung der Klett-Farbzahl erfolgte ohne Bleiche . Die Messung wurde bei einer Konzentration von 5 Gew.-% Aniontensid, pH = 7 und unter Verwendung einer 1 cm-Rundküvette sowie eines Blaufilters (400 bis 465 nm) durchgeführt.

**Tab.2**

| Kenndaten der Beispiele 1 - 5 Prozentangaben als Gew.-% | | | | | | | | |
|---|---|---|---|---|---|---|---|---|
| Bsp. | Edukt | SO₃ Mol | T °C | WAS mEq/g | US % | SO₄²⁻ % | H₂O % | Farbe Klett |
| 1.1 | HK | 1.0 | 30 | 0.032 | 23.7 | 2.6 | 56.9 | 30 |
| 1.2 | HK | 1.0 | 80 | 0.042 | 25.9 | 0.9 | 54.6 | 35 |
| 1.3 | HK | 2.0 | 30 | 0.014 | 6.2 | 1.8 | 84.7 | 41 |
| 1.4 | HK | 2.0 | 80 | 0.028 | 8.4 | 2.0 | 77.0 | 44 |
| 1.5 | HK | 3.0 | 80 | 0.039 | 6.8 | 3.6 | 73.6 | 51 |
| 2.1 | HPK | 1.0 | 30 | 0.035 | 24.1 | 2.4 | 57.1 | 28 |
| 2.2 | HPK | 2.0 | 80 | 0.026 | 8.9 | 2.1 | 76.7 | 39 |
| 3.1 | HP | 2.0 | 80 | 0.049 | 8.0 | 1.1 | 60.4 | 41 |
| 3.2 | HP | 3.0 | 80 | 0.050 | 4.0 | 2.1 | 71.0 | 51 |
| 4.1 | HR | 1.0 | 80 | 0.040 | 9.9 | 1.5 | 50.5 | 39 |
| 4.2 | HR | 2.0 | 80 | 0.047 | 8.0 | 1.7 | 61.9 | 49 |
| 5.1 | HRT | 1.0 | 80 | 0.043 | 9.2 | 0.7 | 50.9 | 32 |
| 5.2 | HRT | 2.0 | 80 | 0.050 | 7.4 | 1.0 | 62.5 | 44 |
| 5.3 | HRT | 3.0 | 80 | 0.048 | 3.9 | 2.2 | 70.1 | 51 |
| Legende: SO₃ = Einsatzmenge Mol SO₃ / Mol Fettsäureester | | | | | | | | |

### Beispiel 6:

Beispiel 1.2 wurde wiederholt. Das rohe Sulfierprodukt wurde jedoch nach Verlassen des Sulfierreaktors und vor der Neutralisation über 30 min bei 80°C einer Nachreaktion unterworfen. Das neutralisierte Produkt mit Nachreaktion zeigte gegenüber dem Produkt ohne Nachreaktion einen verminderten Anteil an unsulfierten Anteilen von 16,7 Gew.-%.

## Patentansprüche

1. Verfahren zur Herstellung von wässrigen Lösungen von Alkali-, Erdalkali-, Ammonium- und/oder Aminsalzen sulfierter Fettsäureglycerinester, **dadurch gekennzeichnet**, daß man Fettsäureglycerinester mit Iodzahlen kleiner 5 mit gasförmigem Schwefeltrioxid in Reaktoren, die nach dem kontinuierlichen Fallfilmprinzip arbeiten, umsetzt und anschließend mit wässrigen Basen neutralisiert.

2. Verfahren nach Anspruch 1, **dadurch gekennzeichnet**, daß man von Mono-, Di- und Triglyceriden oder deren Gemischen mit Iodzahlen kleiner 5 ausgeht, die Fettsäuren mit 8 bis 22 Kohlenstoffatomen enthalten.

3. Verfahren nach mindestens einem der Ansprüche 1 und 2, **dadurch gekennzeichnet**, daß man Fettsäureglycerinester und Schwefeltrioxid im molaren Verhältnis 1 : 0,5 bis 1 : 5 einsetzt.

4. Verfahren nach mindestens einem der Ansprüche 1 bis 3, **dadurch gekennzeichnet**, daß man die Sulfierung bei einer Temperatur von 25 bis 90°C durchführt.

5. Verfahren nach mindestens einem der Ansprüche 1 bis 4, **dadurch gekennzeichnet**, daß man das saure Sulfierprodukt vor der Neutralisation über einen Zeitraum von 5 bis 60 min bei einer Temperatur von 25 bis 90°C einer Nachreaktion unterwirft.

6. Verwendung der nach dem Verfahren nach einem der Ansprüche 1 bis 5 hergestellten wässrigen Lösungen von Alkali-, Erdalkali-, Ammonium- und/oder Aminsalzen sulfierter Fettsäureglycerinester mit Iodzahlen kleiner 5 zur Herstellung oberflächenaktiver Mittel.

## Claims

1. A process for the production of aqueous solutions of alkali metal, alkaline earth metal, ammonium and/or amine salts of sulfonated fatty acid glycerol esters, **characterized in that** fatty acid glycerol esters having iodine values below 5 are reacted with gaseous sulfur trioxide in reactors operating on the continuous falling film principle and are subsequently neutralized with aqueous bases.

2. A process as claimed in claim 1**, characterized in that** mono-, di- and triglycerides or mixtures thereof having iodine values below 5 and containing C₈₋₂₂ fatty acids are used as starting material.

3. A process as claimed in at least one of claims 1 and 2**, characterized in that** fatty acid glycerol ester and sulfur trioxide are used in a molar ratio of 1:0.5 to 1:5.

4. A process as claimed in at least one of claims 1 to 3, **characterized in that** the sulfonation reaction is carried out at a temperature of 25 to 90°C.

5. A process as claimed in at least one of claims 1 to 4, **characterized in that**, before neutralization, the acidic sulfonation product is subjected to an after-reaction for 5 to 60 minutes at a temperature of 25 to 90°C.

6. The use of the aqueous solutions of alkali metal, alkaline earth metal, ammonium and/or amine salts of sulfonated fatty acid glycerol esters having iodine below 5 obtained by the process claimed in any of claims 1 to 5 for the production of surfactants

## Revendications

1. Procédé d'obtention de solutions aqueuses de sels de métal alcalin, de métal alcalino-terreux, d'ammonium et/ou d'amines d'esters de glycérol d'acide gras sulfonatés, caractérisé en ce que l'on fait réagir des esters de glycérol d'acide gras ayant des indices d'iode inférieurs à 5 avec de l'anhydride sulfurique gazeux dans des réacteurs, qui fonctionnent selon le principe du ruissellement en continu et ensuite on neutralise avec des bases aqueuses.

2. Procédé selon la revendication 1 caractérisé en ce que l'on part de mono-, di- et triglycérides ou de leurs mélanges, ayant des indices d'iode inférieurs à 5, qui renferment des acides gras ayant de 8 à 22 atomes de carbone.

3. Procédé selon au moins une des revendications 1 et 2, caractérisé en ce que l'on met en oeuvre l'ester de glycérol d'acide gras et l'anhydride sulfurique dans le rapport molaire de 1:0,5 à 1:5.

4. Procédé selon au moins une des revendications 1 à 3 caractérisé en ce que l'on effectue la sulfonation à une température de 25 à 90°C.

5. Procédé selon au moins une des revendications 1 à 4 caractérisé en ce que l'on soumet le produit de sulfonation avant la neutralisation à une réaction complémentaire pendant un espace de temps de 5 à 60 min à une température de 25 à 90°C.

6. Utilisation des solutions aqueuses produites selon le procédé conforme à l'une des revendications 1 à 5, de sels de métal alcalin, de métal alcalino-terreux, d'ammonium et/ou d'amines d'esters de glycérol d'acide gras sulfonés ayant un indice d'iode inférieur à 5, en vue de la production d'agents tensioactifs.
